# EUROPEAN PATENT SPECIFICATION

(11) **EP 1 987 794 B1**
(45) Date of publication and mention of the grant of the patent: **11.11.2015**
(21) Application number: 08008380.1
(22) Date of filing: 02.05.2008
(51) Int. Cl.: A61B 18/14, A61B 1/018, A61B 17/32, A61B 18/00

(54) **Endoscopic treatment instrument**
Endoskopisches Behandlungsinstrument
Instrument de traitement endoscopique

(30) Priority: 02.05.2007 US 799575
(43) Date of publication of application: 05.11.2008
(73) Proprietor: Olympus Corporation, Shibuya-ku Tokyo (JP)
(72) Inventor: Onishi, Koji, Tokyo (JP)
(74) Representative: von Hellfeld, Axel

(56) References cited:
- WO-A-97/24994
- JP-A- 62 064 355
- US-A- 5 437 662
- US-A1- 2005 010 205

## Description

### BACKGROUND OF THE INVENTION

### Field of the Invention

The present description relates to a natural orifice endoscopic treatment instrument to treat tissue, and a tissue incision method. The invention is as set out in the appended claims.

### Background Art

Natural orifice endoscopic treatment necessary for a patient suffering from a bile duct stone includes feeding a treatment instrument from a flexible endoscope inserted through a duodenam and introducing the treatment instrument into a bile duct through a duodenal papilla. Some treatments include EST (Endoscopic sphincterotomy) for incising a constrictor of the duodenal papilla so that a significant opening for the instrument introduction can be obtained by expanding the opening of the duodenal papilla in advance.

A known treatment instrument suitable for EST is a stylet high-frequency treatment instrument called a needle knife. A needle knife has a stylet electrode that is capable of extending and retracting relative to the tip of a catheter. A conventional needle knife is disclosed by, for example Japanese Unexamined Utility Model (Registration) Application Publication No. S62-50610. The needle knife has a stopper structure at the tip of a catheter so as to regulate a protrusion length of an electrode. US Patent No. 5,536,248 discloses a multi-lumen needle knife having a lumen allowing a guidewire to be inserted therethrough in addition to a lumen having an electrode passing therethrough. An inserted guidewire facilitates an approach of a needle knife to a duodenal papilla and the introduction of another treatment instrument into a bile duct.

A needle knife of this type for use in EST protrudes an electrode from a tip of a catheter and makes the electrode contact a section where one is willing to incise. One must protrude the electrode farther than required for incision since the thickness of a duodenal papilla constrictor differs patient by patient, and since the protrusion length of the electrode is difficult to identify in images obtained through the endoscope. Operating the endoscope to move the electrode incises the constrictor along the path of the electrode movement. Since cholangiography is inoperative in ordinary EST, an endoscopist conducting sphincterotomy along the bile duct predicts directions in the bile duct and the thickness of the bile duct wall based on experience. Therefore, repeating shallow incisions allows the endoscopist to prevent the occurrence of gastrointestinal tract perforation and bleeding. Repeated shallow incisions are carried out with only a part of the electrode tip protruding from the catheter where incision is added according to necessity.

US 2005/0010205 A1 relates to a surgical device for electrosurgery employing high frequency electrical energy, the device comprising a shaft which includes an active electrode. A portion of the electrode is received within an insulating sleeve that extends the entire length of the shaft and thus exposes only an electrode head provided at a distal end of the electrode. The electrode and the insulating sleeve in all operational stages of the surgical device maintain a fixed portion relative to the other components of the shaft.

WO 97/24994 A1 discloses an electrosurgical probe with an electrode unit, the electrode unit comprising a semi-flexible tube shape shaft. A central conductor of an electrode extends through the shaft, a distal end portion of which is constituted by a return electrode, and a ceramic insulation sleeve. An active head of the electrode which is connected to the central conductor protrudes from a distal end of the ceramic insulation sleeve. In a first embodiment of the electrode unit according to WO 97/24994 A1 the central conductor and the active electrode head maintain a fixed position relative to the shaft and the ceramic insulation sleeve. In a second embodiment of the electrode unit according to WO 97/24994 A1 the central conductor, together with the active electrode head, is extensible and retractable relative to the shaft and the ceramic insulation sleeve.

US 5,437,662 discloses an electrosurgical probe comprising an elongate member and a retractable cutting electrode extending through the elongate member. The cutting electrode is coated with an insulating material over its entire length, except for its extreme distal end.

The invention is directed to the objective problem to provide an endoscopic treatment device which prevents an active electrode from being inserted too far into tissue at a point at which a surgeon desires to make an incision.

### SUMMARY OF THE INVENTION

The present invention is as defined in the appended claims that follow. The embodiments, aspects or examples of the present description which do not fall within the scope of said claims are provided for illustrative purposes only and do not form part of the present invention.

An endoscopic treatment instrument according to a first embodiment of the present description includes: a flexible catheter inserted through a channel in an endoscope; a conductive electrode capable of freely extending and retracting through the catheter; and an insulator extending and retracting together with the electrode relative to the catheter so that the insulator isolates, not a distal end portion of the electrode; but the rest of the electrode protruding from the catheter.

A tissue incision method includes: inserting an endoscope into the body of a patient through a natural orifice of a patient; inserting a catheter as an endoscopic treatment instrument through a channel formed in the endoscope; protruding a distal end portion of the electrode together with the insulator from the catheter, the distal end portion of the electrode being exposed and protruding from the insulator; moving together the electrode and the insulator protruding from the catheter; and incising a tissue.

### BRIEF DESCRIPTION OF DRAWINGS

FIG 1 is a schematic view showing a needle knife as an endoscopic treatment instrument.
FIG 2 shows a distal end portion of a catheter of the needle knife in cross section.
FIG 3 is a view on arrow A in FIG 2.
FIG 4 shows the abutted state of the protruding electrode of FIG. 2 making contact with a stopper in cross section.
FIG 5 shows the engagement structure of a first connection section connected to a connector.
FIG 6 shows the needle knife inserted through the endoscope.
FIG 7 shows the electrode extended from the catheter of the needle knife protruding from the endoscope introduced into a duodenal papilla, and abutted to an incision position.
FIG 8 shows how to conduct a first incision.
FIG 9 illustrates a case where an unexpected movement of an object tissue at the incision position changes the positional relationship, i.e., displaces the electrode from the tissue.
FIG 10 illustrates how the positional displacement illustrated in FIG 9 will not promote a deep incision.
FIG 11 illustrates the electrode disposed in preparation of a second incision following the incision of FIG 8.
FIG 12 shows how to conduct the second incision.
FIG 13 illustrates the catheter introduced into the previously incised bile duct and the guidewire passing therethrough.
FIG 14 is a view showing a second connection section attached to the endoscope.

### DETAILED DESCRIPTION OF THE INVENTION

An embodiment of the present description will be hereinafter described in detail with reference to the accompanying drawings.

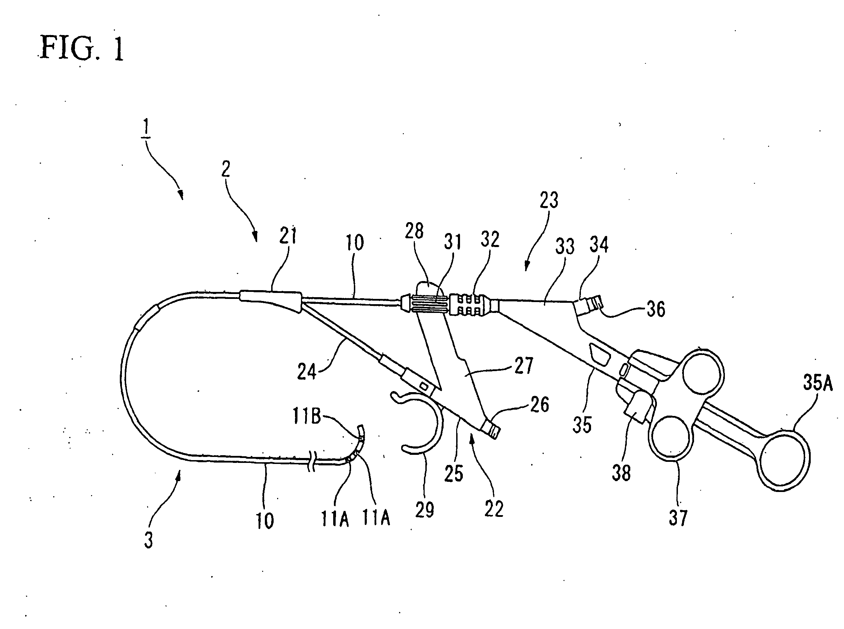

As illustrated in FIG 1, an elongated insertion section 3 extends from an operation section 2, operated by an endoscopist, etc., of a needle knife 1 for use as an endoscopic treatment instrument.

The flexible insertion section 3 constitutes an isulative catheter 10 having three lumens formed therein. Provided to a distal end of the catheter 10 are a mark 11A and a chip 11 B that does not allow X rays to pass therethrough. The distal end portion of the catheter 10 is reduced in diameter and imparted a pre-curve shape for easy approach toward a duodenal papilla. The distal end of the catheter 10 may be without a pre-curve configuration, i.e., with a linear configuration.

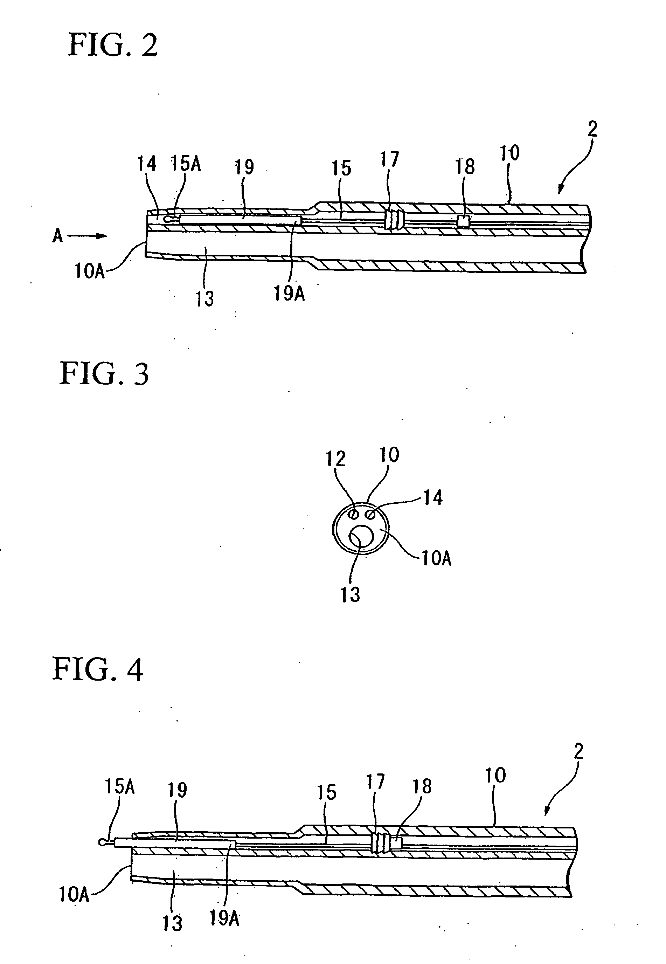

Formed in the catheter 10 are a liquid-feeding lumen 12, a guide lumen 13, and a electrode lumen 14, each having an opening on the distal end surface 10A of the catheter 10 as illustrated in FIGS. 2 and 3. The liquid-feeding lumen 12 is used to inject contrast medium into the bile duct. The guide lumen 13 is used to insert a guidewire therethrough. The guidewire is not shown in the drawings. The electrode lumen 14 is used to pass an electrically-conductive tissue-incising electrode 15 that is capable of extending and retracting therethrough.

A cylindrical stopper 17 is placed in the electrode lumen 14. The stopper 17 is fixed to the wall surface of the incision section 15 due to the engagement of the periphery wedge-shape protrusions thereof. More specifically, the wedges are configured to have a more significant engagement in a direction in which the stopper 17 is pushed toward the distal end. The electrode 15 passing through an inner hole of the stopper 17 extends toward the distal end. An abutment 18 is fixed closer to a proximal end relative to the stopper 17. The outer diameter of the abutment 18 is greater than the inner diameter of the inner hole of the stopper 17 so that the abutment 18 does not hinder the extension and retraction of the electrode 15 in the electrode lumen 14. The electrode 15 can therefore extend until the abutment 18 makes contact with the stopper 17.

The electrode 15 is made from conductive material having elasticity. Except for a part of the electrode 15, the distal end thereof is covered with an insulating-material-made tube 19. The tube 19 is preferably made from a high-melting-point material, namely a fluorine resin because Joule heat is applied during tissue incision. The proximal end 19A of the tube 19 continuously housed in the electrode lumen 14 is never removed from the distal end surface 10A of the catheter 10 while the abutment 18 makes contact with the stopper 17 as illustrated in FIG 4. The tube 19 exposes approximately 1 to 3 mm of the distal end of the electrode 15 to form a incision section 15. The length of the incision section 15A is configured to provide an opening that must be formed at a duodenal papilla at once or several times. The length of the incision section 15A is 3 to 5 mm when the electrode 15 extends until abuts the stopper 17. The most extendable length of 3 to 5 mm facilitates observing the electrode 15 with an image-pickup apparatus of the endoscope disposed at the rear thereof diagonally.

In addition, imparting coloration, e.g., light-blue, to the tube 19 renders the image of the tube 19 obtained through the endoscope distinguishable from other parts in contrast, thereby facilitating observation of the position of the tube 19. The incision depth of the electrode 15 is easily identified by observing the tube 19.

As illustrated in FIG. 1, the operation section 2 is constituted by a first operation section 22, a second operation section 22, and a first branch section 21 joined to the middle of the catheter 10 and branching the operation section 2 into the operation sections 22 and 23. The first operation section 22 extending from the first branch section 21 constitutes of an elongated elastic guidewire tube 24 joined to a guidewire insertion section 25. A part of the guidewire tube 24 in the first branch section 21 is inserted through a guide lumen 13 of the catheter 10. An opening of the guidewire tube 24 is formed on a proximal end of the guidewire insertion section 25. The opening is an insertion port 26 through which the guidewire is inserted.

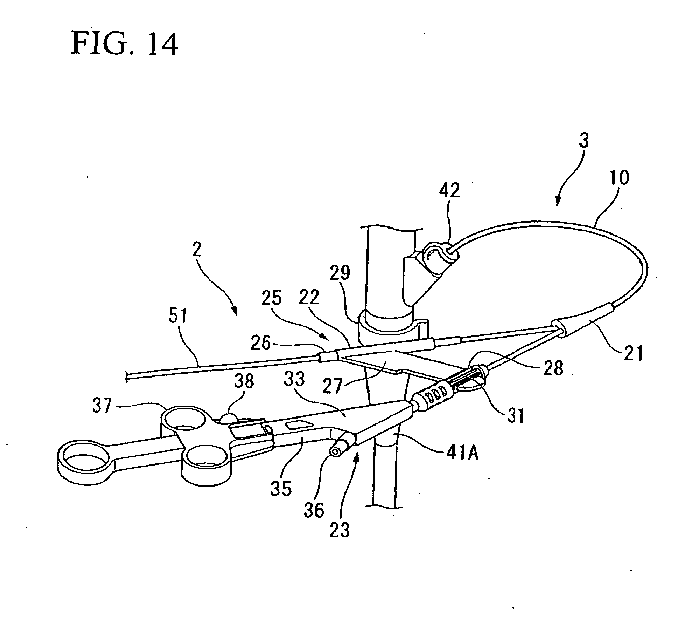

A first connection section 27 extends from a side of the guidewire insertion section 25 toward the second operation section 23. A receiver 28 recesses at a distal end portion of the first connection section 27 and supports the second operation section 23. In addition, a second connection section 29 is disposed on the opposite side of the guidewire insertion section 25. The second connection section 29 is an elastic plate member having a U-shape or C-shape, that allows an endoscope 41, which will be explained later, to be detachably attached to a first connection section 27 via the C-shaped or U-shaped opening as illustrated in FIG 14. The first connection section 27 and the second connection section 29 are disposed on a surface including an axial line of the guidewire insertion section 25.

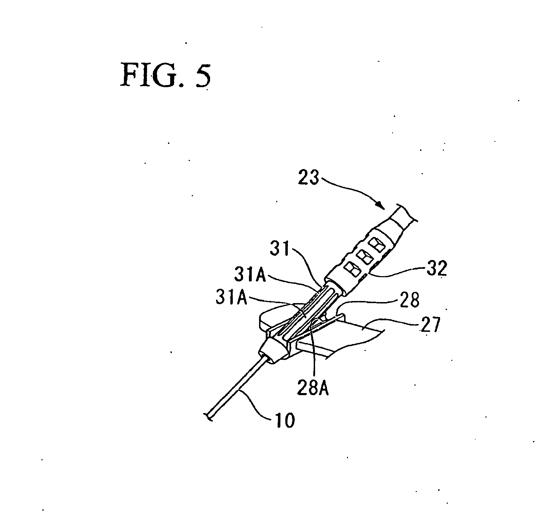

The second operation section 23 constitutes of the catheter 10 passing and extending through the first branch section 21; and a connector 31.joined to an end of the catheter 10. Formed in the connector 31 are protrusions, not shown in the drawings, that engage the catheter 10 to prevent the catheter 10 from moving relative to the connector 31. As illustrated in FIG 5, recesses 3lA are formed on an outer periphery of the connector 31 at regular intervals in a circumference direction. Each recess 3lA extends in the longitudinal direction of the substantially cylindrical connector 31. Provided to the previously described receiver 28 of the first connection section 27 are a plurality of protrusions 28A each entering the recess 31A. The protrusion 28A extending in the longitudinal direction of the recess 31A is of a size configured to be capable of loosely engaging with the recess 31A.

As illustrated in FIG 1, a second branch section 33 is joined to the connector 31 via a deformable section 32 that is capable of freely bending respective to the axial line of the connector 31. The proximal end of the second branch section 33 divides into two sections, i.e., a liquid-feeding section 34 and a sliding section 35. The end of the liquid-feeding section 34 serves as a liquid-feeding cap 36 to which a syringe not shown in the drawings can be connected. The liquid-feeding section 34 has a pipe passing therethrough for use to supply liquids. Respectively the pipe is press-fit watertightly into the liquid-feeding cap 36 and the liquid-feeding lumen 12 of the catheter 10. The sliding section 35 extends diagonally with respect to the axial line of the connector 31. A finger-holding ring 35A is provided to a proximal end of the sliding section 35. A slider 37 capable of extending and retracting in the axial line direction of the sliding section 35 is provided to the sliding section 35 closer to the second branch section 33 relative ring 35A. A plug 38 is provided to the slider 37. The plug 38, wire-connectible to a high frequency power source which is not shown in the drawings, is electrically connected to an electrode 15 passing through the catheter 10. The first operation section 22 and the sliding section 35 in the initial state branch in the same direction as the axial line of the catheter 10. The pre-curve shape imparted to the distal end of the catheter 10 bends in the same direction as those of the first operation section 22 and the sliding section 35.

A manipulation using the needle knife 1 will be explained next.

A side-view endoscope is inserted from a natural orifice of a patient, e.g., a mouth and introduced to a duodenal papilla while observing the interior of the patient with an observation apparatus provided to the distal end of the endoscope. Instead of the mouth, another natural orifice, e.g., a nasal cavity, may be used.

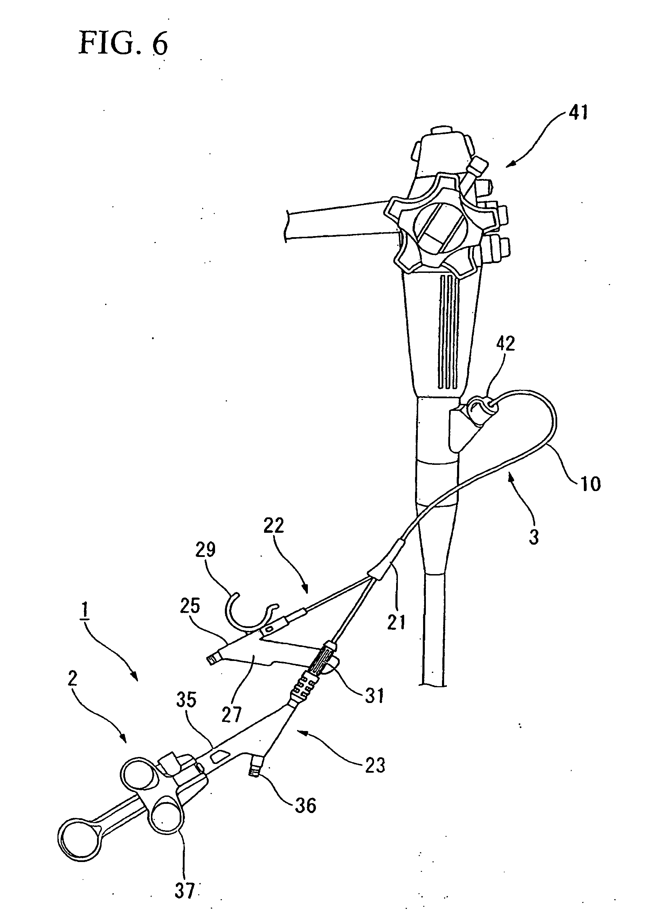

As illustrated in FIG 6, the needle knife 1 is passed from a forceps plug 42 of the endoscope 41 through an operation channel.

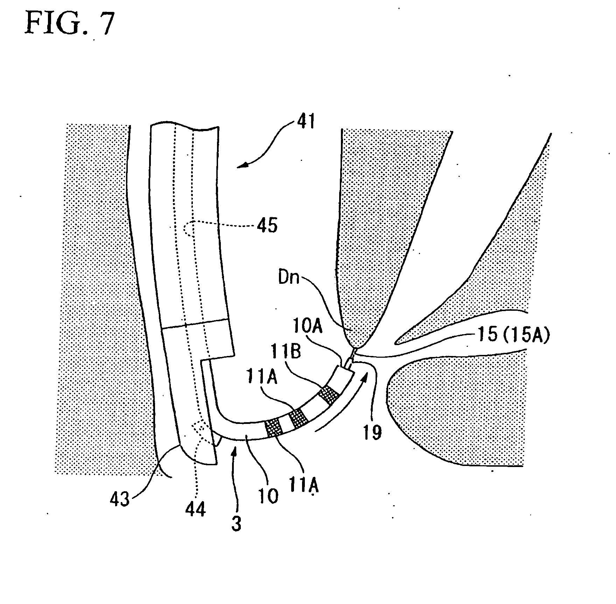

As illustrated in FIG 7, a feed direction of the catheter 10 is adjusted by a raising block 44 provided to a tip cover 43 of the endoscope 41, and the distal end of the catheter 10 is approached to a duodenal papilla Dn.

Holding the slider 37 of the second operation section 23 with fingers and forwarding the slider 37 along the sliding section 35 allow the electrode 15 together with the tube 19 to move, thereby protruding them from the distal end surface 10A of the catheter 10. For example, the length of the incision section 15A exposed from the electrode 15 that is covered by the tube 19 is 1 to 3 mm even if a manipulation moves the electrode 15 forward by 5 mm. The electrode 15 is located downward at a predetermined incision position of the duodenal papilla Dn by a bending operation, i.e., angle-changing manipulations for the endoscope 41 by the endoscopost observing the electrode 15 in an image obtained by the endoscope.

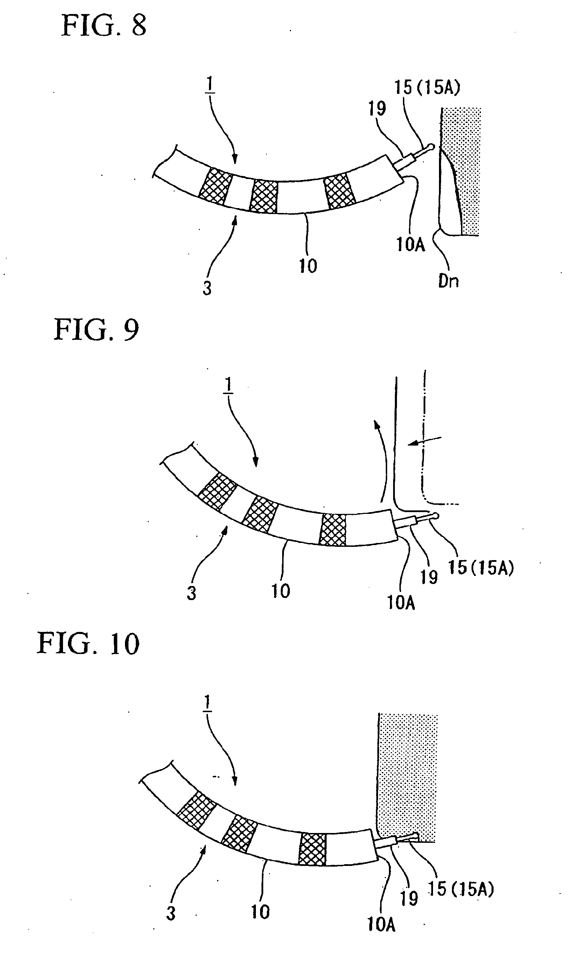

The plug 38 of the slider 37 is connected to the high frequency power source, and high-frequency voltage is charged to the electrode 15. High frequency electric current flowing from the electrode 15 through a tissue toward the ground, not illustrated in the drawings, disposed in the exterior of the patient's body burns and incises the tissue of the duodenal papilla Dn. The catheter 10 is further raised by manipulating the raising block 44. As illustrated in FIG 8, the duodenal papilla Dn is incised tracing the path where the incision section 15A of the electrode 15 has been moved. The high-frequency voltage charging is stopped upon completion of the incision. The incision depth of the duodenal papilla Dn is limited at the length of the incision section 15A or shorter regardless of the feed length of the electrode 15. This is because the portion of the electrode 15 that is covered by the tube 19 never serves for tissue incisions, that is, only the incision section 15A serves therefor.

Sometimes the tissue moves from a position indicated by a broken line to a position indicated by a continuous line while a manipulation is carried out to a patient as illustrated in FIG 9 As illustrated in FIG 10, the portion of the electrode 15 coated by the tube 19, prevented from further penetration, does not incise the tissue deeper than predetermined.

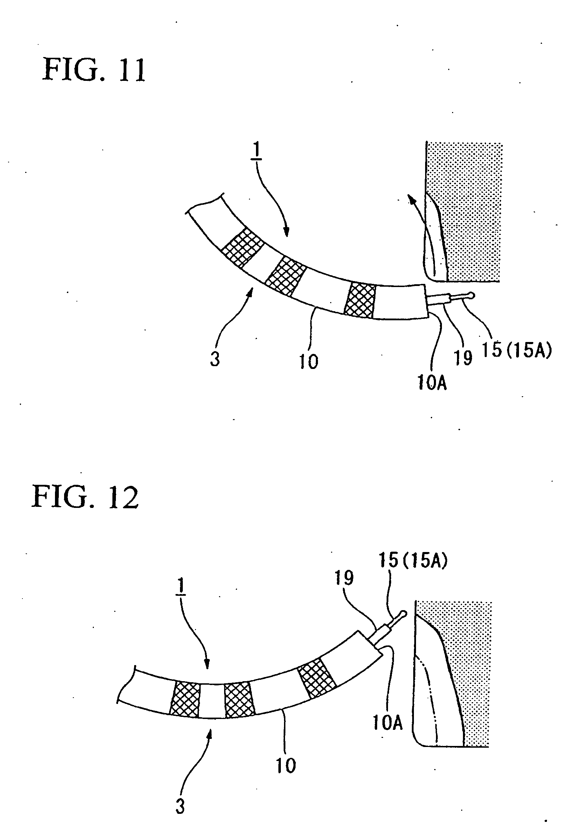

Upon completing the first incision properly, the endoscopist observes the incision part in an image obtained through the endoscope. An additional incision necessitates further forwarding the catheter 10. Alternatively, the electrode 15 should be further forwarded. Although the catheter 10 or the electrode 15 is further protruded, the tube 19 maintains the length of the exposed electrode 15 at 1 to 3 mm that is equal to the length of the incision section 15A. The electrode 15 is located across the once-incised portion as illustrated in FIG 11 by the endoscopost conducting the angle-changing manipulations for the endoscope 41 while observing an image obtained by the endoscope. The electrode 15 is adjusted to be pointed at a position that may further deepen the once-incised portion. A second incision conforming to the first incision path is conducted by recharging high-frequency voltage and raising the catheter 10. The tube 19 having a thickness that enables the tube 19 to enter a space formed in an incised tissue allows the electrode 15 to move smoothly. More specifically, the electrode 15 is approximately 0.2 mm in outer diameter, and the tube 19 is approximately 0.3 mm in outer diameter. The tube 19 should be; at maximum, 0.1 mm in wall thickness. As illustrated in FIG 12, the second incision added after the first incision further deepens the incision. The incisions as such are repeated until obtaining necessary incision depth or a necessary opening.

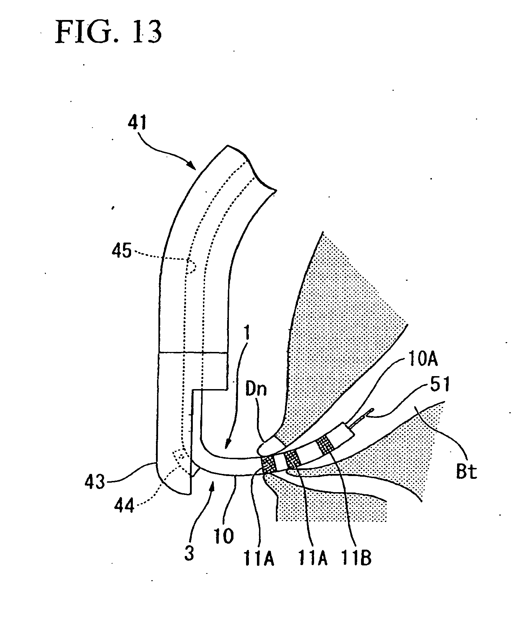

The slider 37 is drawn back upon obtaining an opening having a significant size by incising from the duodenam to the bile duct. The electrode 15 together with the tube 19 is housed in the catheter 10. As illustrated in FIG 13, subsequently the distal end portion of the catheter 10 is inserted from the partly-incised and enlarged opening of the duodenal papilla Dn through the bile duct Bt. The guidewire 51 is inserted from an insertion port 26 at the proximal end of the guidewire insertion section 25. The guide wire 51 is forwarded from the guidewire tube 24 into the guide lumen 13 of the catheter 10, and fed into the bile duct Bt from the distal end of the catheter 10. Contrast medium stored in a syringe attached to the liquid-feeding cap 36 is fed from the opening of the distal end of the liquid-feeding lumen 12 into the bile duct Bt through a liquid-feeding lumen 12. The contrast medium fed into the bile duct Bt increases visibility of the bile duct Bt under X-ray radiation.

Subsequently the needle knife 1 is retracted from the bile duct Bt while the guidewire 51 remains in the bile duct Bt. Furthermore, another instrument, e.g., a basket forceps is inserted through the bile duct Bt to conduct necessary treatments in place of the needle knife 1 retracted from the operation channel 45 of the endoscope 41. The guidewire S guides and introduces this new instrument into the bile duct Bt. Upon completing the treatment, the instruments and the guidewire 51 are retracted from the patient followed by the endoscope 41.

In one case here in the present embodiment, the endoscopist may manipulate the endoscope 41 while a supporter uses the operation section 2 in turn to manipulate the needle knife 1 in accordance with the endoscopist's instructions. In contrast, in another case, the connection between the connector 31 and the first connection section 27 is released when the endoscopist and the supporter share the operation of the needle knife 1 or the endoscopist solely operates the needle knife 1. As illustrated in FIG 14; a bend stop portion 41A of the endoscope 41 closer to the distal end relative to the forceps plug 42 is engaged to the second connection section 29. The guidewire insertion section 25 is fixed to the endoscope 41. In particular, insertion of the guidewire 51 by the endoscopist can be facilitated by disposing the insertion port 26 of the guidewire 51 by disposing the insertion port 26 for the guidewire 51 to be at the right-hand side with respect to the endoscopist who holds the endoscope 41 by his (or her) left hand. Since the operations for the endoscope 41 and the guidewire 51 can be carried out solely by the endoscopist, the insertion of the guidewire 51 into a narrow tubular path can be facilitated. These operations, e.g.; extension and retraction of the electrode 15 and liquid supply carried out solely by the endoscopist, can be facilitated since the second operation section 23 is joined to the endoscope 41 via the first and second connection sections 27 and 29.

In addition, adjustably rotating the connector 31 around the receiver 28 of the first connection section 27 permits easy operation thereof for the endoscopist. Adjusting the direction of connector 31 relative to the receiver 28 is easy due to loose engagement between them. Maintaining the directions thereof is also easy even if they are untouched.

The connection between the first connection section 27 and the connector 31 is released in a case where the endoscopist manipulates the guidewire 51 and the supporter handles extension and retraction of the electrode 15 and the liquid supply. Operation by the supporter is easy since the sliding section 35 and the liquid-feeding section 34 can be disposed separately from the endoscope 41 having disposition freedom, i.e., the flexible catheter 10.

The present embodiment allows the length of the electrode 15 contributing to incision to be maintained at a constant length, thereby providing easy control for the incision depth even if the observation of the electrode 15 in an image obtained through the endoscope necessitates the protrusion thereof more than that of the incision. Extreme caution must be taken in conventional configurations where a deeper incision than planned is likely to be caused by an unexpected movement of a section to be incised because the protrusion of an electrode is longer than the required incision length. The needle knife 1 according to the present invention prevents such unexpected incisions, thereby reducing the endoscopist's stress. The needle knife 1 can solve the problem that delicate treatments were difficult in the conventional configurations since incisions with an electrode having a constant protrusion length require providing additional pushing force to a catheter into a previously-incised portion.

The stopper 17 placed in the catheter 10 prevents excessive protrusion of the electrode 15. Furthermore, an unexpected incision can be avoided since the maximum protrusion of the electrode 15 exposes only the distal end portion of the electrode 15 from the tube 19. The protrusion length of the electrode 15 even bending in the middle of the elongated catheter 10 can be controlled since the stopper 17 is disposed at the distal end portion of the catheter 10. The tube 19 is provided to only a portion of the electrode 15 toward a distal end relative to the stopper 17. The catheter 10 having a thinner diameter and sufficient flexibility than can be obtained in a case where a catheter is disposed over an entire length of the electrode lumen 14.

Since the length of the incision section 15A of the electrode 15 exposed from the tube 19 is 1 to 3 mm, an incision or two can provide the necessary opening regardless of a personal difference in thickness of the portion to be incised. Certainly incisions may be in more than three times.

In addition, the catheter 10 may be of a single lumen structure without the guide lumen 13 or the liquid-feeding lumen 12. The catheter 10 may be constituted of the electrode lumen 14 and one of the lumens 12 and 13.

The distal end portion of the needle knife 1 may be in a spatula shape or hook shape in place of the needle-shaped distal end of the stylet high-frequency treatment instrument that has been explained with respect to the electrode 15. In these cases, the tube 19 covers everything except the distal end portion. The exposure length of the electrode 15 is adjusted so that an incision or two can provide the necessary opening.

The needle knife 1 and the manipulation thereof may be employed in ESD (Endoscopic Submucosal Dissection). The endoscope 41 may be inserted from another natural orifice such as a nasal cavity.

Although the preferred embodiment has been explained, note that the present invention is not limited to the above descriptions but is limited only by the appended claims.

The insulative member applied to the needle knife 1 may be coating as long as it imparts insulation to the electrode 15 in place of the tube 19 that was referred to in the previous explanation.

The invention is as defined in the appended claims.

## Claims

1. An endoscopic treatment instrument (1) adapted to perform an endoscopic sphincterotomy comprising:
a flexible catheter (10) configured to be inserted through a channel in an endoscope, the flexible catheter (10) comprising an electrode lumen (14);
a conductive electrode (15) capable of freely extending and retracting through the electrode lumen (14);
a stopper (17) having an inner hole and being fixed to an inner wall surface of the electrode lumen (14), wherein the conductive electrode (15) passes through the inner hole; and
an abutment (18) which is fixed to the electrode (15) and is disposed proximal relative to the stopper (17), wherein
the outer diameter of the abutment (18) is greater than the inner diameter of the inner hole of the stopper (17) so that the abutment (18) does not hinder the extension and retraction of the electrode (15) in the electrode lumen (14),
an insulator (19) configured to extend and retract together with the electrode (15) relative to the flexible catheter (10), wherein
the insulator (19) covers the electrode (15) so that a distal end of the electrode (15) protrudes from a distal end of the insulator (19), the insulator (19) isolating the rest of the electrode (15) protruding from the flexible catheter (10); wherein
the insulator (19) is configured to expose 1 to 3 mm of the distal end of the electrode (15) to form an incision section, and wherein
a proximal end of the electrode (15) protrudes from a proximal end of the insulator (19).

2. The endoscopic treatment instrument (1) according to Claim 1, wherein the thickness of the insulator (19) is 0.1 mm or lower.

3. The endoscopic treatment instrument (1) according to Claim 2, wherein the insulator (19) is made of a fluoro-resin.

4. The endoscopic treatment instrument (1) according to Claim 2, wherein the stopper (17) is adapted to regulate the protrusion length of the electrode (15).

5. The endoscopic treatment instrument (1) according to Claim 4, wherein the insulator (19) is disposed at only a portion between the distal end (15A) of the electrode (15) and the stopper (17).

6. The endoscopic treatment instrument (1) according to Claim 1, wherein the flexible catheter (10) is a multi-lumen catheter.

## Patentansprüche

1. Endoskopisches Behandlungsgerät (1), ausgebildet zum Ausführen einer endoskopischen Sphinkterotomie und umfassend:
einen flexiblen Katheter (10), ausgebildet um durch einen Kanal in ein Endoskop eingeführt zu werden, der flexible Katheter (10) umfasst ein Elektrodenlumen (14);
eine leitende Elektrode (15), die imstande ist, sich durch das Elektrodenlumen (14) frei auszudehnen und zurückzuziehen;
ein Verschluss (17), der ein inneres Loch aufweist und an einer inneren Wandoberfläche des Elektrodenlumen (14) fixiert ist, wobei die leitende Elektrode (15) durch das innere Loch passt; und
eine Führung (18), welche an der Elektrode (15) fixiert und nahe dem Verschluss (17) angeordnet ist, wobei
der äußere Durchmesser der Führung (18) größer als der innere Durchmesser des inneren Lochs des Verschlusses (17) ist, so dass die Führung (18) nicht die Ausdehnung und das Zurückziehen der Elektrode (15) in dem Elektrodenlumen (14) verhindert,
ein Isolator (19) ausgebildet um sich zusammen mit der Elektrode (15) relativ zu dem flexiblen Katheter (10) auszudehnen und zurückzuziehen, wobei
der Isolator (19) die Elektrode (15) umgibt, so dass ein distales Ende der Elektrode (15) von dem distalen Ende des Isolators (19) hervorsteht, wobei
der Isolator (19) den Rest der Elektrode (15), der von dem flexiblen Katheter (10) hervorsteht, isoliert; wobei
der Isolator (19) ausgebildet ist zum Freigeben von 1 bis 3 mm des distalen Endes der Elektrode (15), um einen Schnittbereich zu formen, und wobei
ein proximales Ende der Elektrode (15) von einem proximalen Ende des Isolators (19) hervorsteht.

2. Endoskopisches Behandlungsgerät (1) nach Anspruch 1, bei dem die Dicke des Isolators (19) 0,1 mm oder weniger beträgt.

3. Endoskopisches Behandlungsgerät (1) nach Anspruch 2, bei dem der Isolator (19) aus Fluorharz hergestellt ist.

4. Endoskopisches Behandlungsgerät (1) nach Anspruch 2, bei dem der Verschluss (17) zum Regulieren der hervorstehenden Länge der Elektrode (15) ausgebildet ist.

5. Endoskopisches Behandlungsgerät (1) nach Anspruch 4, bei dem der Isolator (19) nur an einem Abschnitt zwischen dem distalen Ende (15A) der Elektrode (15) und dem Verschluss (17) angeordnet ist.

6. Endoskopisches Behandlungsgerät (1) nach Anspruch 1, bei dem der flexible Katheter (10) ein Multi-Lumen-Katheter ist.

## Revendications

1. Instrument de traitement endoscopique (1) adapté pour réaliser une sphincté-rotomie endoscopique et comprenant :
un cathéter flexible (10) configuré pour être inséré à travers un canal dans un endoscope, le cathéter flexible (10) comprenant une lumière d'électrode (14) ;
une électrode conductrice (15) capable de s'étendre et de se rétracter librement à travers la lumière d'électrode (14),
un élément d'arrêt (17) comportant un trou interne et étant fixé à une surface de paroi interne de la lumière d'électrode (14), dans lequel l'électrode conductrice (15) passe à travers le trou interne ; et
une butée (18) qui est fixée à l'électrode (15) et est disposée proximale par rapport à l'élément d'arrêt (17), dans lequel
le diamètre externe de la butée (18) est supérieur au diamètre interne du trou interne de l'élément d'arrêt (17), de sorte que la butée (18) n'empêche pas l'extension et la rétraction de l'électrode (15) dans la lumière d'électrode (14),
un isolant (19) configuré pour s'étendre et se rétracter conjointement avec l'électrode (15) par rapport au cathéter flexible (10), dans lequel
l'isolant (19) couvre l'électrode (15) de sorte qu'une extrémité distale de l'électrode (15) fait saillie depuis une extrémité distale de l'isolant (19), l'isolant (19) isolant le reste de l'électrode (15) faisant saillie depuis le cathéter flexible (10) ; dans lequel
l'isolant (19) est configuré pour exposer 1 à 3 mm de l'extrémité distale de l'électrode (15) pour former une section d'incision, et dans lequel
une extrémité proximale de l'électrode (15) fait saillie depuis une extrémité proximale de l'isolant (19).

2. Instrument de traitement endoscopique (1) selon la revendication 1, dans lequel l'épaisseur de l'isolant (19) est de 0,1 mm au moins.

3. Instrument de traitement endoscopique (1) selon la revendication 2, dans lequel l'isolant (19) est composé de résine fluorée.

4. Instrument de traitement endoscopique (1) selon la revendication 2, dans lequel l'élément d'arrêt (17) est adapté pour réguler la longueur de saillie de l'électrode (15).

5. Instrument de traitement endoscopique (1) selon la revendication 4, dans lequel l'isolant (19) est disposé uniquement au niveau d'une partie entre l'extrémité distale (15A) de l'électrode (15) et l'élément d'arrêt (17).

6. Instrument de traitement endoscopique (1) selon la revendication 1, dans lequel le cathéter flexible (10) est un cathéter à lumières multiples.
